# EUROPÄISCHE PATENTSCHRIFT

(11) **EP 0 673 904 B1**
(45) Veröffentlichungstag und Bekanntmachung des Hinweises auf die Patenterteilung: **25.11.1998**
(21) Anmeldenummer: 95103373.7
(22) Anmeldetag: 09.03.1995
(51) Int. Cl.: C07C 15/46, C07C 5/333, B01J 8/06

(54) **Verfahren zur Dehydrierung von Ethylbenzol**
Process for the dehydrogenation of ethylbenzene
Procédé pour la déshydrogénation d'éthylbenzènes

(30) Priorität: 16.03.1994 DE 4408889
(43) Veröffentlichungstag der Anmeldung: 27.09.1995
(73) Patentinhaber: BASF AKTIENGESELLSCHAFT, 67056 Ludwigshafen (DE)
(72) Erfinder: Deimling, Axel, Dr., D-67434 Neustadt (DE); Ruppel, Wilhelm, Dr., D-67227 Frankenthal (DE); Behling, Uwe, D-67240 Bobenheim-Roxheim (DE); Biedenkapp, Dieter, Dr., D-67133 Maxdorf (DE)

(56) Entgegenhaltungen:
- DD-A- 231 924
- DE-A- 2 229 132
- DE-B- 1 290 130
- US-A- 4 229 603

## Beschreibung

Zur technischen Dehydrierung von Ethylbenzol zu Styrol sind verschiedene Verfahren bekannt (Ullmanns Encyklopädie der technischen Chemie, 4. Aufl., Bd. 22, S. 298). Praktisch angewendet werden das sog. adiabate Verfahren, das meistens in zwei adiabatisch betriebenen, in Reihe geschalteten Radialstromreaktoren mit Zwischenaufheizung oder Zwischendampfeinspeisung betrieben wird, und das sog. isotherme Verfahren, bei dem ein Rohrbündelreaktor mit indirekter Wärmeübertragung verwendet wird. Auch dabei wird zusätzlich Wasserdampf verwendet.

Es ist außerdem bekannt, daß die Raum-Zeit-Ausbeute des isothermen Verfahrens in einfachen Rohrbündelreaktoren, bei denen lediglich die Rohre mit Katalysator gefüllt sind, durch Vorschalten (DE-A-1 059 437) oder Nachschalten (DE-A-1 290 130) einer "adiabaten" Katalysatorschicht verbessert werden kann. In DE-A-34 43 118 ist auch eine Kombination einer vorgeschalteten und einer nachgeschalteten adiabatisch betriebenen Reaktionszone mit einem Rohrbündelreaktor beschrieben.

Die Einspeisung einer hohen, stark vorerhitzten Dampfmenge zur Auslösung einer adiabaten Reaktion hat jedoch den Nachteil, daß gerade der Vorzug des "isothermen" Verfahrens, nämlich der Betrieb bei niedrigem Dampf/Ethylbenzol-Verhältnis, aufgegeben und auf einen Teil der an sich möglichen Raum-Zeit-Ausbeute verzichtet wird.

Es ist Aufgabe der Erfindung, die Raum-Zeit-Ausbeute gegenüber isotherm betriebenen Rohrbündelreaktoren weiter zu steigern, wobei auf die Durchführung einer adiabaten Vorreaktion verzichtet werden kann, jedoch nicht muß.

Erfindungsgegenstand ist ein Verfahren zur Dehydrierung von Ethylbenzol zu Styrol unter Wärmezufuhr durch einen Wärmeträger an einem im wesentlichen in einem Rohrbündel angeordneten Dehydrierkatalysator, wobei man den Wärmeträger mindestens im Bereich des Rohrbündels im Gegenstrom zum Reaktionsgemisch führt.

Das Reaktionsgemisch wird also erfindungsgemäß im Rohrbündel indirekt mit Heizgas unter Anwendung des Gegenstromprinzips erhitzt. Während der Durchleitung des gasförmigen Reaktionsgemisches durch den Rohrbündelreaktor setzt mit der Aufheizung allmählich die Reaktion ein. Die Aufheizung reicht soweit, daß am Ende des Rohrbündelreaktors schon ein wesentlicher Teilumsatz stattgefunden hat. Mit der Gegenstrombeheizung kann aber gleichzeitig eine Überhitzung des Reaktionsgemisches vorgesehen werden, so daß noch eine ausreichende Restenergie für eine adiabate Nachreaktion vorhanden ist. Auf diese Weise kann der Anteil der adiabaten Nachreaktion an der Gesamtreaktion wesentlich crößer sein als bei dem in DE 1 290 130 beschriebenen Verfahren und spielt sich zudem im selektivitätsgünstigen Bereich geringsten Druckes ab. Dem adiabat betriebenen Anlagenteil kommt damit eine ähnliche Bedeutung zu wie dem beim "adiabaten" Verfahren üblichen zweiten Reaktor.

Erfindungsgemäß wird somit erreicht, daß alle Vorteile der bekannten Verfahren - hohe Selektivität des adiabaten Verfahrens, niedriges Dampf/Ethylbenzol-Verhältnis durch indirekte Wärmezufuhr beim isothermen Verfahren - miteinander kombiniert sind. Das erfindungsgemäße Verfahren zeichnet sich außerdem dadurch aus, daß drei Verfahrensschritte, nämlich Aufheizung, erste Teilreaktion im Rohrbündel und Nachreaktion in einem einzigen Apparat ablaufen können.

Für das nachstehend beschriebene Verfahrensbeispiel wird die folgendermaßen aufgebaute Versuchsanordnung verwendet:

Ein 5500 mm langes Einzelrohr (1), das einen Innendurchmesser von 78 mm hat, wie es in Rohrbündeln von technischen Reaktoren verwendet wird, ist von einem Mantelrohr (2) umgeben, das oben und unten Anschlüsse (2a, b) für Zu- und Ableitung einen Wärmeträgers hat, sitzt auf einem 1200 mm langen rohrförmigen Reaktionsraum (3) mit einem Durchmesser von 163 mm, in den an der Verjüngungsstelle unten ein Rost (3a) eingebaut ist (vgl. Figur). In verschiedener Höhe sind Probenahmestutzen (I-VII) angeordnet.

Der gesamte Innenraum wird mit einem handelsüblichen Katalysator (G84C der Fa. Südchemie) gefüllt. Der untere Reaktionsraum weist keinen Mantel auf und dient der adiabaten Nachreaktion.

Wasserdampf und Ethylbenzol werden von oben zugeführt und das unten austretende Reaktionsgemisch kondensiert und untersucht.

Es ergibt sich folgendes:

### Beispiel

Bei einem Dampf/Ethylbenzol-Verhältnis von 1,25 kg/kg und einer Gesamtquerschnittsbelastung im Rohrteil von 240 kmol/m²h (Dampf + Ethylbenzol) wird am Ende der adiabaten Nachschicht ein Ethylbenzol-Umsatz von 64 % und eine Styrol-Selektivität von 95,5 mol-% erzielt. Der Druck am Reaktorende ist 0,5 bar_{abs}. Die Temperatur des Katalysators und des Reaktionsgemisches am Apparateeintritt ist 420°C, am Ende des Rohrbündels 620°C und am Ende der Nachreaktionsschicht 585°C.

Es versteht sich, daß die Erfindung nicht auf den Reaktorbetrieb von oben nach unten (bzw. unten nach oben für den Wärmetauscher) beschränkt ist, sondern ebensogut mit einer umgekehrt arbeitenden Anordnung verwirklicht werden kann.

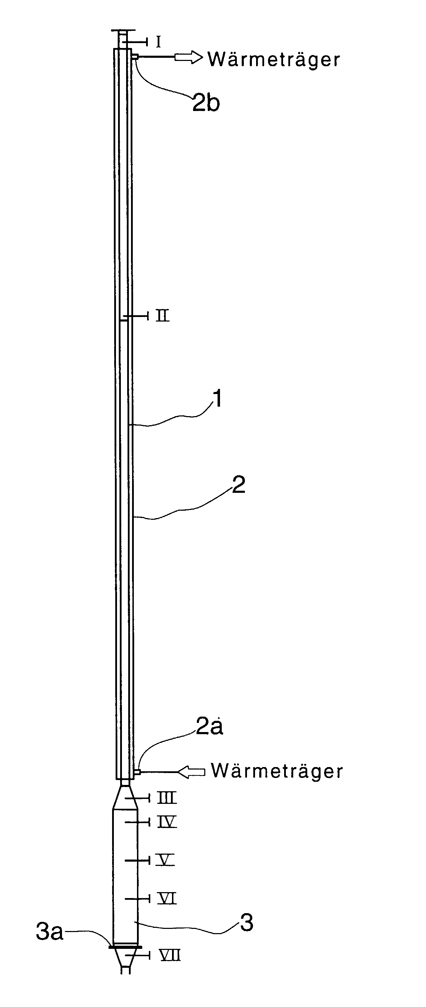

## Patentansprüche

1. Verfahren zur Dehydrierung von Ethylbenzol zu Styrol an einem in einem Rohrbündel angeordneten Dehydrierkatalysator und einem weiteren, nachgeschalteten Reaktionsraum zur adiabaten Nachreaktion, dadurch gekennzeichnet, daß das Reaktionsgemisch aus Wasserdampf und Ethylbenzol im Rohrbündel indirekt durch einen Wärmeträger im Gegenstrom von 420°C auf 620°C erhitzt wird.

## Claims

1. A process for the dehydrogenation of ethylbenzene to styrene on a dehydrogenation catalyst which is located in a tube bundle and a further, downstream reaction chamber for subsequent adiabatic reaction, wherein the reaction mixture consisting of steam and ethylbenzene is heated from 420°C to 620°C in the tube bundle indirectly by means of a countercurrent heat transfer medium.

## Revendications

1. Procédé pour la déshydrogénation d'éthylbenzènes en styrène à un catalyseur de déshydrogénation situé dans un faisceau de tuyau et à une chambre de réaction supplémentaire placée en aval pour une réaction ultérieure adiabatique, caractérisé en ce que le mélange de réaction de vapeur d'eau et d'éthylbenzènes est chauffé de manière indirecte dans le faisceau de tuyau à l'aide d'un fluide caloporteur à contre-courant de 420° à 620°.
